# EUROPEAN PATENT APPLICATION

(11) **EP 2 535 072 A2**
(43) Date of publication of application: **19.12.2012**
(21) Application number: 12180240.9
(22) Date of filing: 07.06.2005
(51) Int. Cl.: A61M 5/178

(54) **Subcutaneous infusion devices**

(30) Priority: 07.06.2004 US 577658 P
(62) Divisional of application: 05757206.7
(71) Applicant: C. R. BARD, INC., Murray Hill, NJ 07974 (US)
(72) Inventor: Barron, William R., Riverton, UT 84065 (US); Burnside, Eddie K., Bountiful, UT 84010 (US); Hamatake, Bret, Grantsville, UT 84029 (US); Powers, Kelly B., North Salt Lake, UT 84054 (US)
(74) Representative: HOFFMANN EITLE

(57) **Abstract**

A subcutaneous infusion device, including an infusion set and conduit, the infusion set including a cannula and a hub. The cannula may include a coil component that is helically arranged around a longitudinal axis to define a lumen and a tubing component that is associated with the coil component and may take on the shape of the outer surface thereof. The cannula may include a proximal guide to assist in the insertion of a needle therethrough. Both integral and attachable infusion set and conduit combinations are described, as well as a sterile package and insertion mechanism.

## Description

### PRIORITY

This application claims the benefit under 35 U.S.C. § 119(e) of U.S. Provisional Application No. 60/577,658, filed June 7, 2004, which is expressly incorporated by reference as if fully set forth herein.

### BACKGROUND

Infusion sets are known in the art for delivering a medication to a patient at a subcutaneous site. Such infusion sets, which generally include at least an integral tubing portion and hub portion, are connected to other components of an infusion pump system following subcutaneous placement at a designated site. The most common use for infusion sets as described herein is for the delivery of insulin to a diabetic patient. In the United States alone, it is estimated that there are one million Type 1 diabetics that require intensive insulin therapy to sustain life and reduce diabetes related complications. Patients classified as Type 1 diabetics do not themselves produce insulin and must therefore obtain the necessary amounts from an external source. While there are different ways in which Type 1 diabetics can receive their daily requirement of insulin, one method is through the use of an infusion pump system, which is capable of delivering a constant dose of insulin to the patient throughout the day.

A typical infusion pump system includes a programmable infusion pump that is compact and can be carried by a patient, an infusion set that provides subcutaneous access to the patient, and a conduit attaching the infusion pump to the infusion set. The infusion pump generally includes a small motor, driven by a battery, as well as a microprocessor to regulate the rate and timing of the insulin delivery to the infusion set assembly. The infusion set generally includes a fine grade cannula made of metal or plastic that perforates the epidermis of a patient and delivers insulin subcutaneously, following insertion thereof by an insertion device and attachment to the infusion pump. The cannula is generally offered in a 90-degree configuration and a variable angle configuration. The conduit generally includes plastic tubing that is fluidly connected to the cannula, having an attachment mechanism for attachment to the infusion pump. Insertion of the infusion set involves placing a needle with an attached proximal hub through the cannula of the infusion set, loading the needle hub into an insertion mechanism, and firing the infusion set/needle combination into a designated site. This process is generally performed by the patient at home, due to the necessity of altering the access site every few days as the site becomes saturated.

While improvements to infusion pumps have been significant throughout the more than twenty years of use thereof by diabetic patients, little technological advancement has taken place with respect to infusion sets. In particular, infusion set patency, ease of use, sterility, safety and user comfort are examples of areas that have gone largely unaddressed, despite the growing number of complaints by users. Regarding infusion set patency, for example, the cannulas used in the majority of currently-sold systems may kink or otherwise become closed to fluid delivery, which is a potentially life-threatening problem (a user can experience shock within eight hours of discontinuation of insulin delivery). Kinking and/or closure may occur for a number of reasons, such as insertion procedure, infusion set placement site, user activity, adhesive failure (resulting in delamination and shearing), etc. Unfortunately, due to the relatively slow rate of delivery of insulin by the infusion pump in most circumstances and/or the unreliability of pump overpressure alarms, a kink or closure in the cannula may not be discovered until it is too late (i.e., the patient goes into shock).

### BRIEF SUMMARY

Accordingly, embodiments regarding the design and manufacture of an insertion set and/or conduit attaching the insertion set to an infusion pump are provided herein. In one embodiment, an infusion set includes a coil-reinforced cannula that is resistant to kinking and therefore is useful for maintaining patency of the infusion set. A cannula including a coil-reinforced portion also reduces cannula profiles, which results in patient comfort. In one embodiment of a coil-reinforced cannula, an integral proximal funnel is provided to assist in the guiding of an insertion needle through the cannula. In one embodiment of a cannula for an infusion set, an internal lumen is provided with a cross-sectional shape to prevent kinking thereof when bent. In another embodiment of a cannula for an infusion set, perforations or apertures are provided in a wall thereof along a length of a cannula body portion to facilitate distribution of medication to the patient.

In one embodiment, an insertion set and conduit assembly are separate and attachable, such that the conduit is attached to the infusion set following insertion of the infusion set into a desired site of a user's body. In one embodiment of an attachable assembly, a safety feature is provided to indicate to a clinician or user if and when the infusion set has been disconnected from the conduit. In another embodiment of an attachable assembly, an activity cover is provided to permit periodic safe removal of the conduit from the infusion set. In a particular embodiment of an infusion set, child-friendly features are incorporated. In one embodiment, an insertion set is integral with the conduit, the device incorporating features to facilitate use thereof.

In a particular embodiment of a method for manufacturing a cannula, a heat shrink method is employed to combine a tubing material with a coil component. In another embodiment of a method for manufacturing a cannula, an RF encapsulated tip technique is employed. In a further embodiment of a method for manufacturing a cannula, an RF infiltrated technique is employed. In yet another embodiment of a method for manufacturing a cannula, an injection molded infiltrated technique is employed. In still another embodiment of a method for manufacturing a cannula, an injection molded encapsulated technique is employed. In a further still embodiment of a method for manufacturing a cannula, dip coating technique is employed. In another embodiment of a method for manufacturing a cannula, a heat shrink infiltrated technique is employed.

In one embodiment, a sterile package/insertion device is provided for delivery and insertion of the infusion set that preserves sterility before, during and after insertion of the infusion set. In another embodiment of a sterile package/insertion device, an encased infusion set is inserted into a user without the needle tip of an insertion needle becoming exposed to the user, thereby preventing accidental needle sticks.

These and other embodiments, features and advantages of the present invention will become more apparent to those skilled in the art when taken with reference to the following more detailed description of the invention in conjunction with the accompanying drawings that are first briefly described.

### BRIEF DESCRIPTION OF THE DRAWINGS

FIG. 1 is a cross-sectional view of a distal end of an embodiment of a cannula.

FIG. 2 is a cross-sectional view of a distal end of another embodiment of a cannula.

FIG. 3 is a cross-sectional view of a distal end of yet another embodiment of a cannula.

FIG. 4 is a cross-sectional view of a distal end of still another embodiment of a cannula.

FIG. 5 is a perspective view of one embodiment of a coil component of a cannula in isolation.

FIG. 6 is a perspective view of another embodiment of a coil component of a cannula in isolation.

FIG. 7 is a perspective view of an embodiment of a perforated cannula.

FIG. 8 is an enlarged view of the distal end of the cannula of FIG. 7.

FIG. 9 is a perspective view of another embodiment of a perforated cannula.

FIG. 10 is an enlarged view of the distal end of the cannula of FIG. 9.

FIG. 11 is a longitudinal cross-sectional view of the distal end of the cannula shown in FIG. 10.

FIGS. 12-17 are cross-sectional views of different cannula embodiments, each showing examples of different possible lumen shapes thereof.

FIG. 18A is a perspective view of an embodiment of an attachable infusion set and conduit.

FIG. 18B is a cross-sectional view of the infusion set and conduit of FIG. 18A, taken along line B-B.

FIG. 18C is an end view of the infusion set and conduit of FIG. 18A.

FIGS. 19A-C illustrate the infusion set and conduit of FIG. 18A in three different stages of attachment of the infusion set to the conduit.

FIG. 20 is another embodiment for an attachable infusion set and conduit.

FIG. 21 is an enlarged cross-sectional view of the attached infusion set and conduit of FIG. 19C.

FIG. 22 is a perspective view of one embodiment of an integral infusion set and conduit prior to insertion of an insertion device.

FIG. 23 is an enlarged view of FIG. 22 with the needle of the insertion device inserted through the cannula of the integral infusion set and conduit.

FIG. 24 is a partial enlarged view of FIG. 23.

FIG. 25 is a cross-sectional view of FIG. 24.

FIG. 26A is a perspective view of an embodiment of an attachable infusion set and conduit.

FIG. 26B is a top view of the attachable infusion set and conduit of FIG. 26A.

FIG. 26C is a cross-sectional view of the attachable infusion set and conduit of FIG. 26B, taken along line C-C.

FIG. 27A is a perspective view of an embodiment of an activity cover for an inserted infusion set.

FIG. 27B is a top view of the activity cover and inserted infusion set of FIG. 27A.

FIG. 27C is a cross-sectional view of the activity cover and inserted infusion set of FIG. 27B, taken along line C-C.

FIG. 28 is a cross-sectional view of a die assembly used for manufacturing a cannula, with a core-pin and assembly positioned therein, at one stage of a manufacturing process.

FIG. 29 is a cross-sectional view of the die assembly embodiment of FIG. 28, with a core-pin and assembly positioned therein, at a stage of the manufacturing process subsequent to the stage shown in FIG. 28.

FIG. 30 is a cross-sectional view of the die assembly embodiment of FIG. 28, with a core-pin and assembly positioned therein, at a stage of the manufacturing process subsequent to the stage shown in FIG. 29.

FIG. 31 is a cross-sectional view of another embodiment of a die assembly used for manufacturing a cannula, with a core-pin and assembly positioned therein, at one stage of the manufacturing process.

FIG. 32 is a cross-sectional view of the die assembly embodiment of FIG. 31, with a core-pin and assembly positioned therein, at a stage of the manufacturing process subsequent to the stage shown in FIG. 31.

FIG. 33 is a cross-sectional view of the die assembly embodiment of FIG. 31, with a core-pin and assembly positioned therein, at a stage of the manufacturing process subsequent to the stage shown in FIG. 32.

FIG. 34A is a perspective view of one embodiment of a sterile package and insertion mechanism with an infusion set loaded therein.

FIG. 34B is a cross-sectional view of the sterile package and insertion mechanism of FIG. 34A.

FIGS. 35A-B are cross-sectional views of the sterile package and insertion mechanism of FIG. 34A with the sterile barrier removed.

FIGS. 36A-36B are cross-sectional views of the embodiment shown in FIG. 34A, following deployment of the insertion device.

FIGS. 37A-37B are cross-sectional views of the embodiment shown in FIG. 34A, following retraction of the insertion device from the infusion set.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

### Infusion Set and Conduit

The infusion set described herein is advantageous for a number of reasons, one of which is a non-kinking cannula design, which combines tubing made of materials such as polyolefin, FEP, Pebax, PET, etc., with a very small diameter coil component, having a diameter in the range of approximately 0.0005 in. to 0.010 in., and in some embodiments in a range of approximately 0.0015 in. to 0.005 in. Embodiments of the cannula design, in addition to having the advantage of kink-resistance, are also very small in profile, which is important to patients that typically insert an infusion set into their bodies two to three times per week.

In the embodiments described herein, it should be appreciated that numerous variations are possible with respect to the configuration of the coil component and tubing. For example, in some embodiments, the coil component is continuous, being arranged helically about a longitudinal axis, while in others, the coil component is individual rings or other coil shapes that are positioned along a longitudinal axis of the cannula. In some embodiments, the coil pitch (spacing) is either open (spaces between turns of the coil or individual coil components) or closed (no spaces between turns of the coil or individual coil components) along the entire length of the cannula. In other embodiments, the coil pitch is partially open and partially closed along selected lengths. The cross-sectional shape of the coil component may include circular, quadrilateral, triangular or other cross-sectional shapes. Moreover, the coil component may include metal or other rigid materials, such as engineering plastics (e.g., polyetheretherketon (PEEK)), carbon fiber, glass, etc. In a specific embodiment, the coil component can include a wire.

The coil component in some embodiments is embedded within a tubing wall, and in others is partially or completely internal or external thereof. In some embodiments, the coil component extends into the tip of the cannula, while in others the distal end of the coil component is proximal to the tip. The proximal end of the coil component in some embodiments is shaped in the form of a funnel or needle guide to assist in the insertion of a needle therethrough. Moreover, in some embodiments, the cannula includes perforations or apertures in selected locations, which may be similarly or differently sized. Further, depending on the desired configuration of the cannula, numerous different manufacturing and tipping processes are employed in selected embodiments. Thus, while specific examples and embodiments are described herein, it should be appreciated that many different design possibilities exist for each, as well as methods of manufacture, some of which will be described in more detail below.

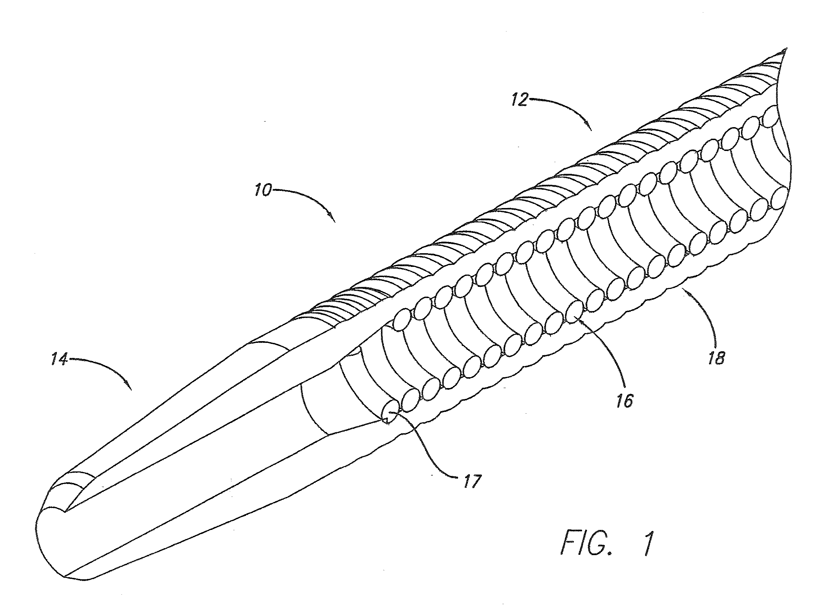

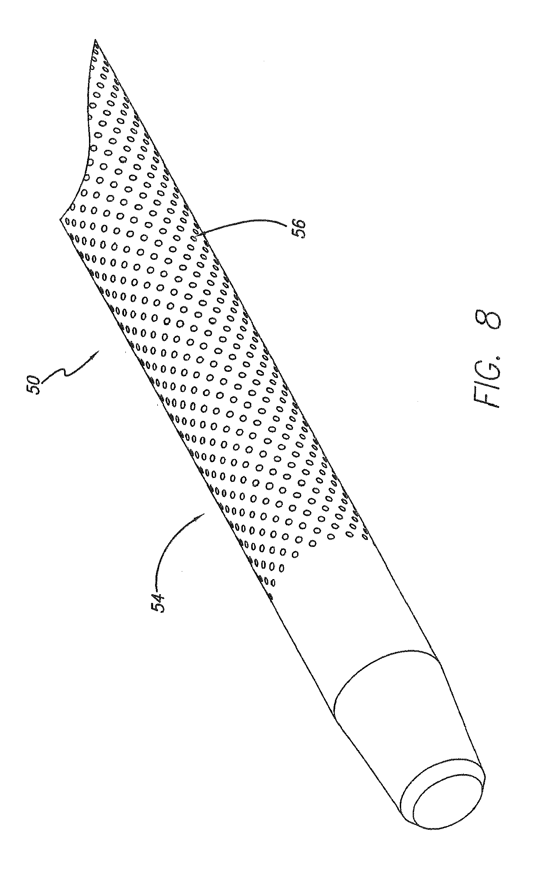

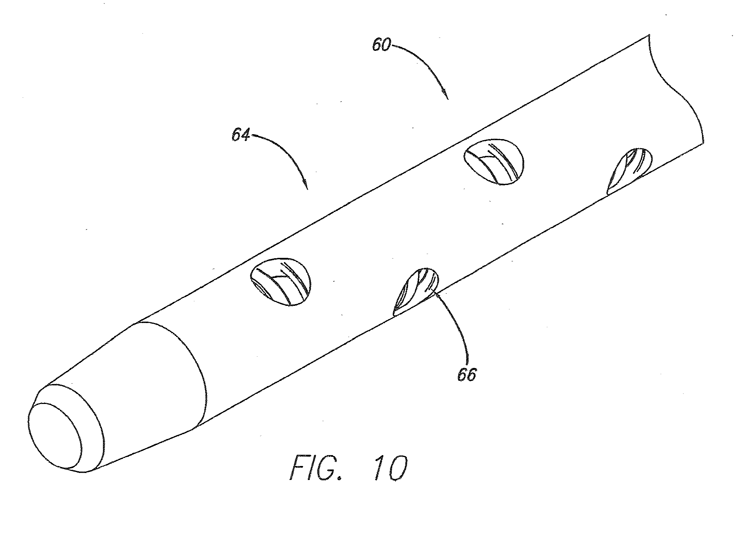

FIGS. 1-27 illustrate various views of different embodiments of a cannula for an infusion set. The cannula generally includes a distal tip section, a body section, and a proximal section. FIG. 1 illustrates one embodiment of a cannula, showing an enlarged view of a distal tip section 14 and a distal end of a body section 12 of a cannula 10. The cannula 10 includes a coil component 16 and a tubing component 18, the coil component 16 being coiled in a closed pitch configuration, covered on an outer surface thereof by the tubing component 18. The cannula 10 can include a corrugated outer surface that assumes the outer surface profile of the closed pitch coil component 16. The distal end 17 of the coil component 16 does not extend into the cannula tip in this embodiment. Cannula 10 is created, for example, by using either a heat shrink or injection molded encapsulated technique as will be explained in more detail below.

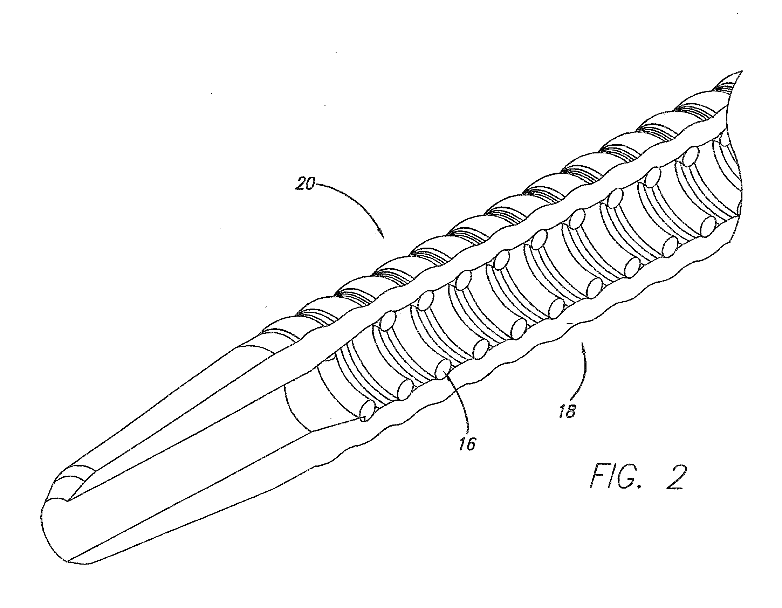

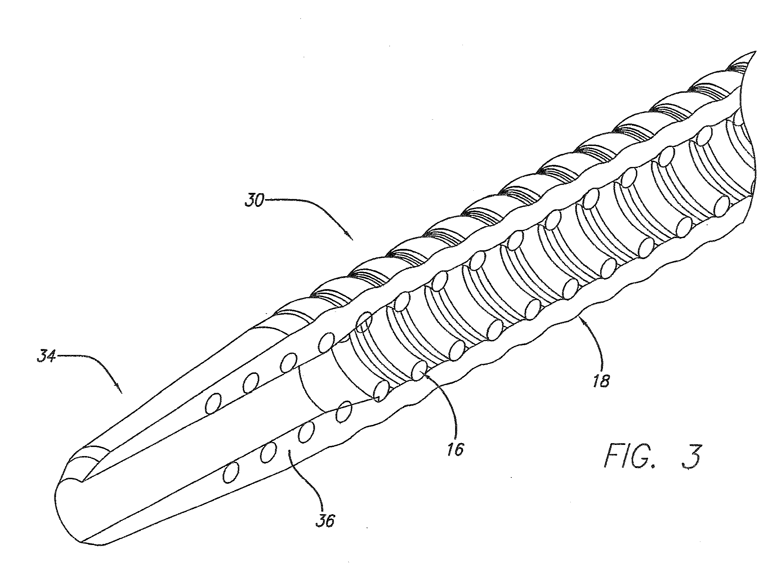

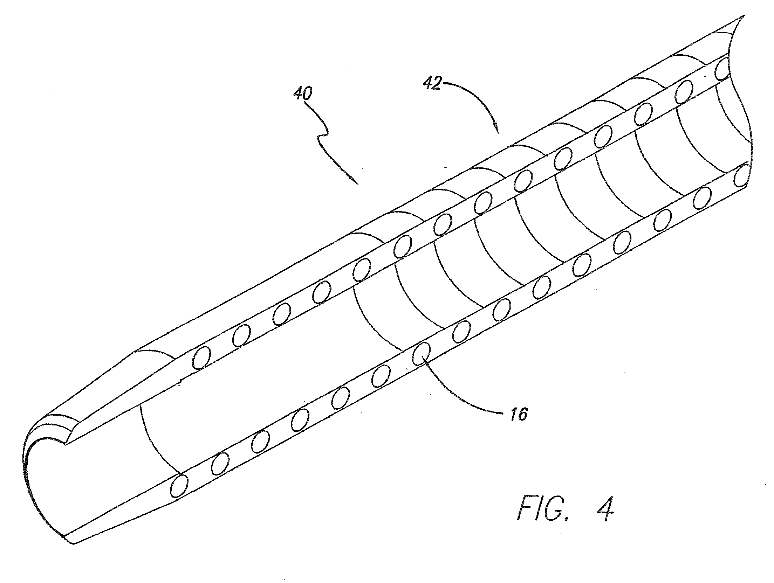

FIG. 2 illustrates another embodiment of a cannula, similar to cannula 10 but instead including a coil component 16 that is coiled in an open pitch configuration. The tubing component 18 of cannula 20 fills in the gaps created by openings between windings of the coil component 16, creating a corrugated outer surface. Cannula 20 is created, for example, by a heat shrink technique as will be explained in more detail below. Cannula 30, shown in FIG. 3, also has a coil component 16 with an open pitch configuration, but in this embodiment, the coil component 16 extends into the cannula tip 34 and is embedded in a wall 36. In addition, the outside surface of cannula 30 is smooth, rather than corrugated. Cannula 30 is created, for example, by an RF encapsulated technique as will be explained in more detail below. FIG. 4 shows another embodiment with an open pitch configuration and a smooth outer cannula wall. However, the coil component 16 of cannula 40 in FIG. 4 is embedded in the wall of the cannula body section 42. Cannula 40 is created, for example, by an RF infiltrated or injection molded infiltrated technique as will be explained in more detail below.

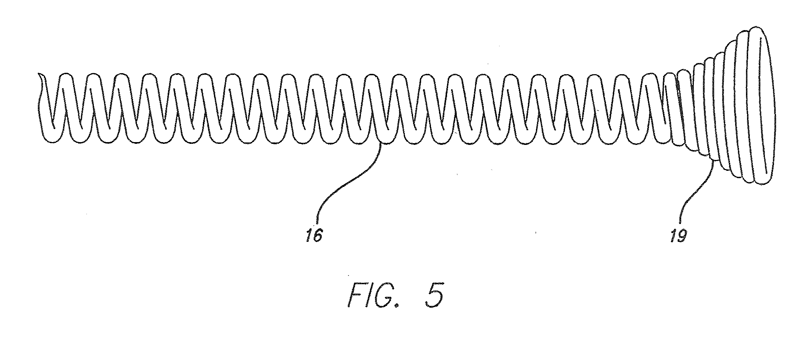

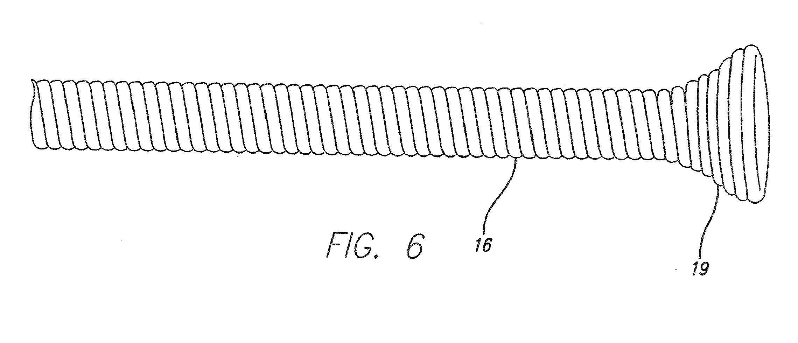

FIGS. 5 and 6 show a proximal section 19 of the coil component 16 used in creating cannulas 10, 20, 30 and 40. The proximal section 19 of the coil component 16 is shaped in the form of a funnel or guide to aid the insertion of a needle through the cannula. While the coil component 16, as shown in FIGS. 5, is coiled in an open pitch configuration along the body section thereof, the proximal section 19 has a closed pitch configuration to ensure that the needle passes safely through the body section and does not penetrate through the tubing wall. This funnel configuration may be incorporated into the proximal end of the coil component 16 of any of the embodiments of FIGS. 1-4. FIG. 6 is an embodiment in which both the body and proximal section are coiled in a closed pitch configuration.

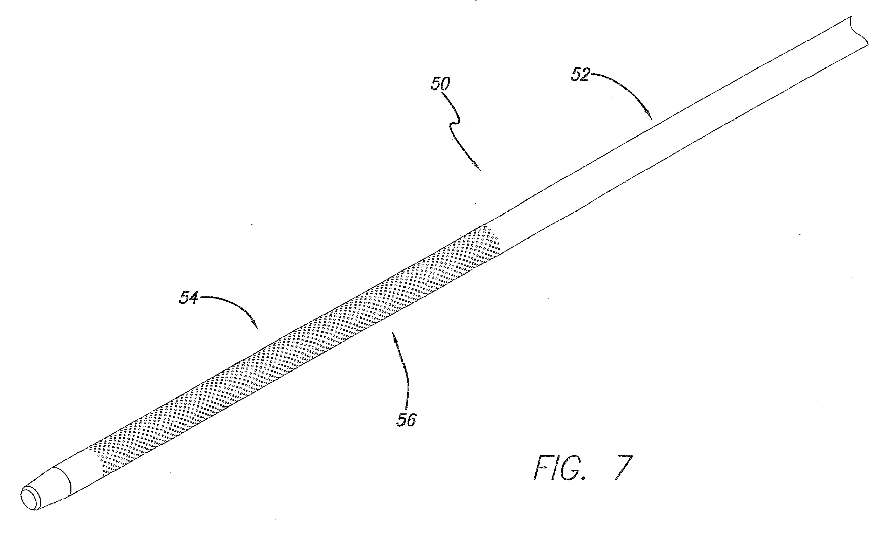

FIGS. 7-11 illustrate another design possibility for an embodiment of a cannula, namely, perforations or apertures in the tubing wall to permit fluid delivery therethrough. The perforations or apertures may be positioned in various configurations (e.g., offset, aligned, around the entire circumference of the cannula, around only a portion of the circumference of the cannula, etc.), along certain specified lengths of the cannula (e.g., along one length of the cannula, along two lengths of the cannula separated by non-perforated lengths, along a distal section, along a proximal section, etc.), and have a diameter generally in the range of approximately 0.0005 in to 0.020 in., and in some embodiments in the range of approximately 0.001 in to 0.012 in. As is known to one of ordinary skill in the art, the perforations may be fashioned in the cannula following the formation thereof by techniques such as laser drilling, skiving or ablation (which laser techniques can perforate the tubing component without disturbing the coil component), or alternatively by techniques such as mechanical punching, drilling or skiving. The advantages of having perforated sections in the cannula, include, for example, enhanced absorption of medication at the delivery site, a potential increase of indwelling time at a given delivery site, etc.

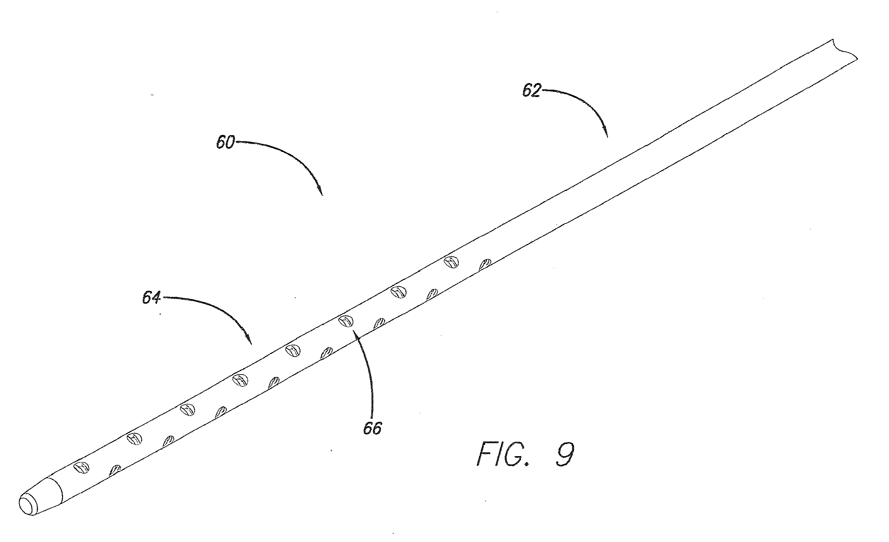

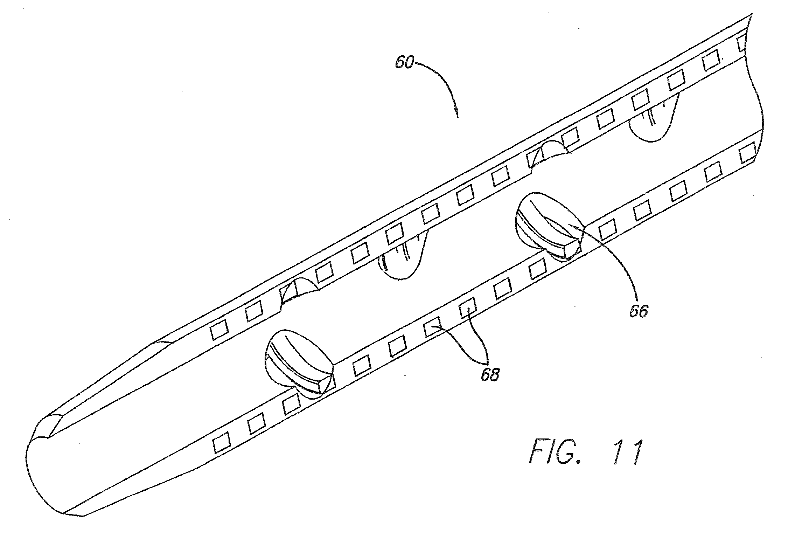

FIGS. 7-8 illustrate one embodiment of a perforation design in a cannula 50, where small perforations or apertures 56 are created in a tubing wall at a distal end 54 of a body section 52 of the cannula 50, the perforations or apertures 56 extending a length along the distal end 54 of the body section 52. The length of the perforation section may range from a small fraction of the distal end 54 of the cannula 50 to a substantial length of the body section 52; however, the length of the perforation section generally should not extend proximally along the body section 52 such that accumulation of medication on the skin surface results from infusion thereof through the cannula 50. The perforations or apertures 56 are shown in FIGS. 7-8 to have a relatively small diameter and are aligned in alternating offset circumferential rows around the entire circumference of the cannula 50. FIGS. 9-11 illustrate a cannula 60, including perforations or apertures 66 positioned in a distal end 64 of a body section 62. The perforations or apertures 66 in cannula 60 are larger than the perforations or apertures 56 in cannula 50. Also, in cannula 60, the perforations or apertures 66 are positioned in offset longitudinal rows. FIG. 11 illustrates a cross-sectional view of the cannula 60, showing a coil component 68 with an open pitch configuration, a rectangular cross-sectional shape and embedded within the wall of the tubing (although it should be appreciated that other coil configurations and shapes are certainly possible).

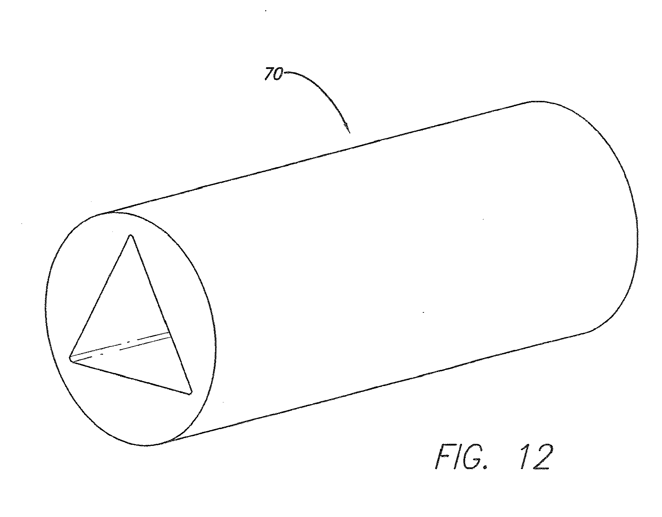

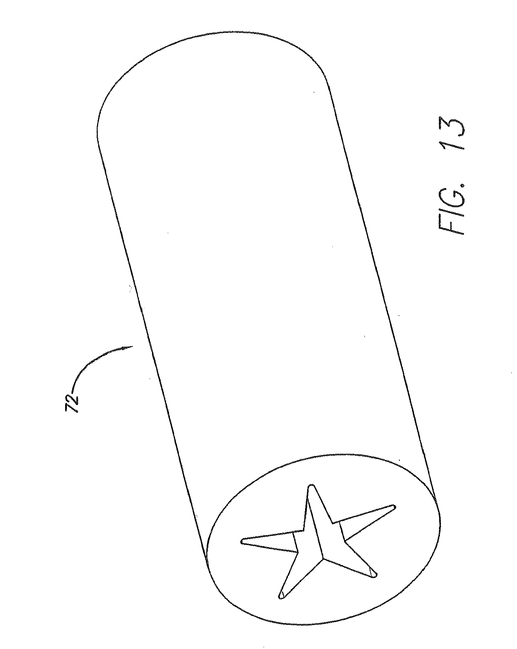

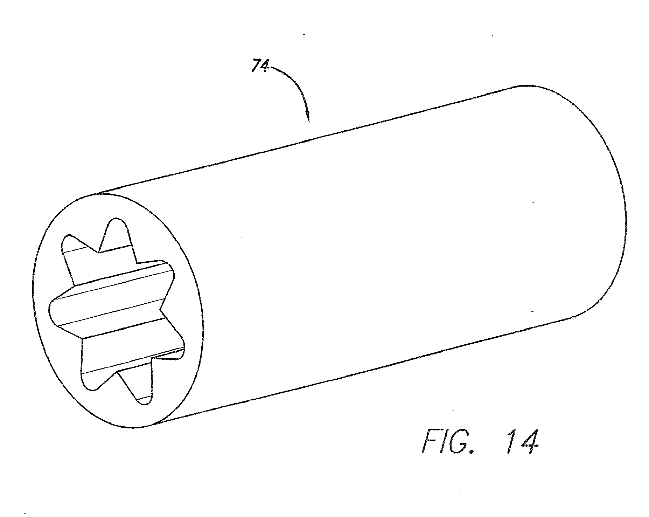

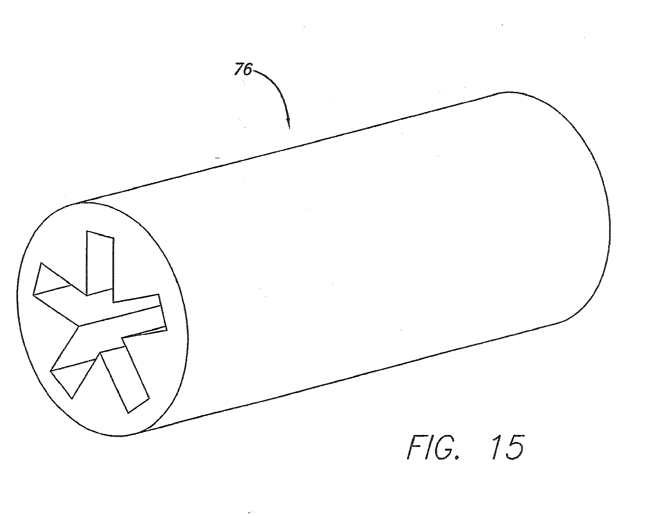

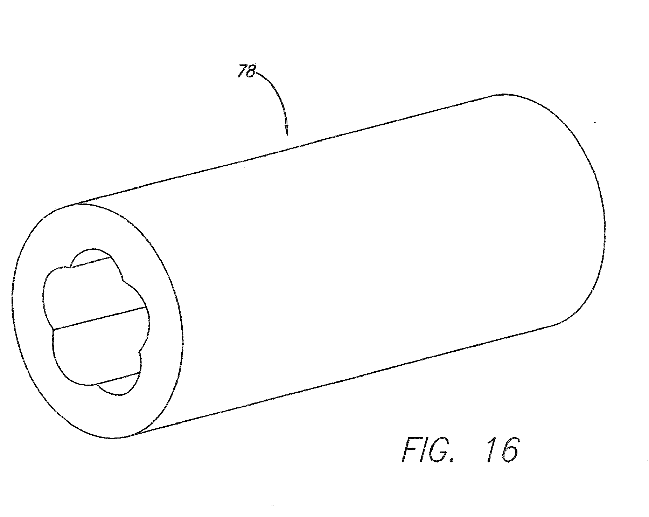

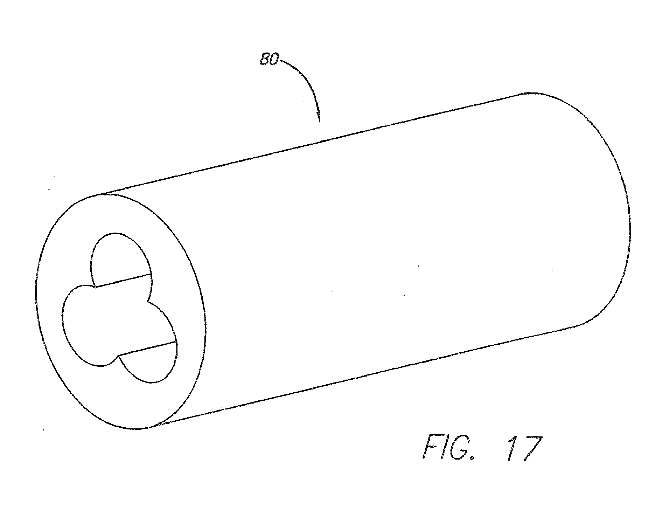

FIGS. 12-17 illustrate another embodiment of a cannula, in which the patency of the cannula is maintained through internal geometry of the tubing lumen. FIGS. 12-17 illustrate cannula lumens having various different non-kinking cross-sectional shapes, which remain open for fluid flow therethrough even when the cannula is bent or twisted. FIG. 12 illustrates a cannula 70 with a triangular-shaped lumen; FIG. 13 illustrates a cannula 72 with a star-shaped lumen; FIG. 14 illustrates a cannula 74 with a septa-lobed lumen; FIG. 15 illustrates a cannula 76 with an asterisk-shaped lumen; FIG. 16 illustrates a cannula 78 with a penta-lobed lumen, the lobes being broader than those in the lumen of cannula 74; and FIG. 17 illustrates a cannula 80 with a triple-lobed lumen, the lobes being deeper than those in the lumen of cannula 78. Of course, while certain particular cross-sectional shapes are illustrated, it should be appreciated that other shapes are contemplated and within the scope of this invention. It should also be appreciated that the depth and breadth, for example, of sections of the lumen cross-sectional shapes can be greater or smaller than illustrated, as one of ordinary skill in the art would appreciate.

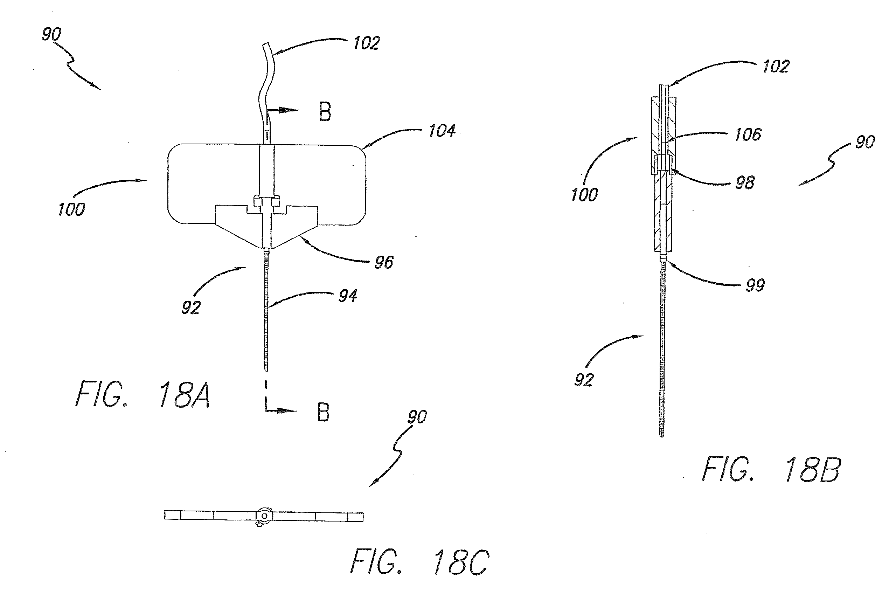

FIGS. 18-21 illustrate an embodiment of an attachable infusion set and conduit assembly that differs from a standard type of integral infusion set and conduit assembly generally known in the art. The embodiment illustrated includes an infusion set and conduit that are initially separate, but which are attachable following insertion of the infusion set into the body. FIG. 18A is a perspective view of an attachable system 90, including an infusion set component 92 with a coil-reinforced cannula 94 (i.e., a cannula having a coil component as described above with a proximal funnel portion) and an infusion set hub or wing 96, and a conduit component 100 including a conduit tubing 102 and a conduit hub or wing 104. FIG. 18B illustrates a cross-sectional view of the attachable system 90, showing a cannula 106 positioned at a distal end of the conduit wing 104 for penetrating a septum 99 positioned in a proximal section of the infusion set wing 96. The septum 99, as known to one of ordinary skill in the art, is a piece of re-sealable material placed in a fluid flow path to prevent back flow of fluid when an inserted instrument is withdrawn. In one embodiment, the septum 99 is made of silicone. FIG. 18C is an end view of the attachable system 90. While the infusion set hub 96 and conduit hub 104 in this embodiment are wing-shaped, designed to offer a large surface area to facilitate attachment to the skin of a patient (e.g., using tape or other adhesives), other hub configurations are possible and are contemplated herein.

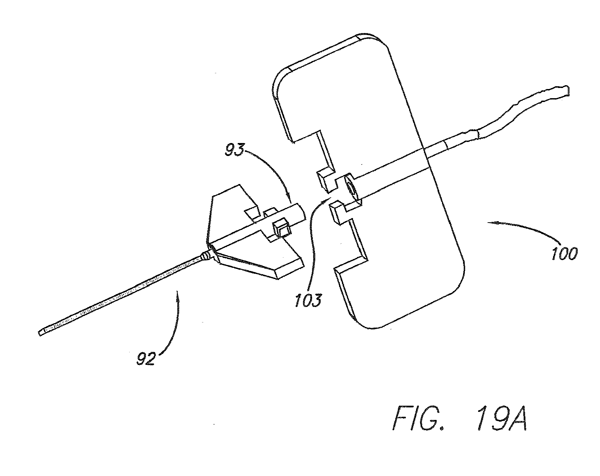

FIGS. 19A-19C illustrate the attachment of the conduit 100 to the infusion set 92, showing a three-step process, following insertion of the infusion set 92 into a user's body. In FIG. 19A, a proximal member 93 of the infusion set 92 is aligned with a distal opening 103 of the conduit 100 along a common axis. In this particular embodiment, the proximal member 93 is in the form of a "t" shape, including a proximal cylindrical section and extending protrusions. The distal opening 103 of the conduit wing 104 includes an annular region into which the proximal cylindrical section is inserted, as well as a recess shaped to accommodate the remaining section of the proximal member 93 as shown in FIG. 19A. When the proximal member 93 is inserted into the distal opening, it is first rotated approximately 90 degrees (FIG. 19A illustrates the beginning of the rotation of the proximal member 93) so that the protrusions of the proximal member do not prevent passage through the entry point to the distal opening 103.

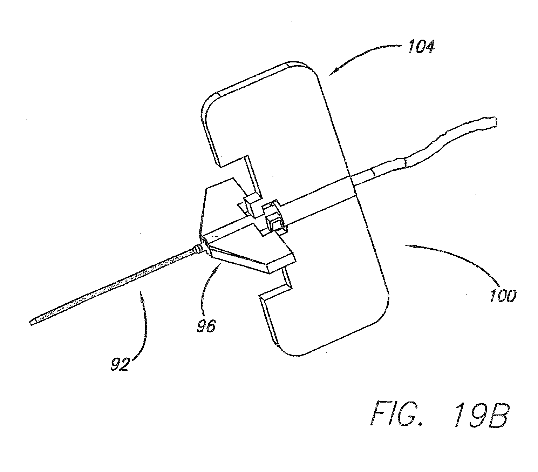

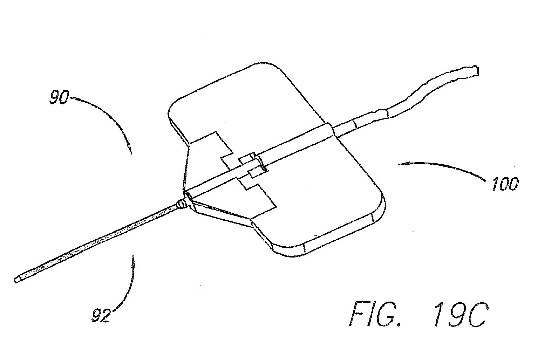

In FIG. 19B, the cannula 106 of the conduit 100 is inserted through the septum 98 of the infusion set 92, such that the infusion set wing 96 is approximately perpendicular to the conduit wing 104. During or after the proximal member 93 is inserted into the annular region of the conduit wing 104 and the cannula 106 is inserted through the septum 98, it is rotated back to its parallel position with respect to the infusion set wing 96 as shown in FIG. 19C, which acts to lock the infusion set 92 to the conduit 100. It is noted here that the geometry of the distal edge of the conduit wing 104 is shaped to mate with the proximal edge of the infusion set wing 96 in order to provide a locking interface. While the locking feature shown comprises protrusions on the proximal member 93 of the infusion set 92 locking into recesses in the conduit 100, it should be appreciated that various mechanical and other types of locking features, such as a threaded engagement, a snapping engagement (e.g., snap ring, circlip, etc.), etc., could equally be utilized and are within the scope of the present invention. Moreover, in one embodiment, rather than a detachable assembly, the locking mechanism can be permanent.

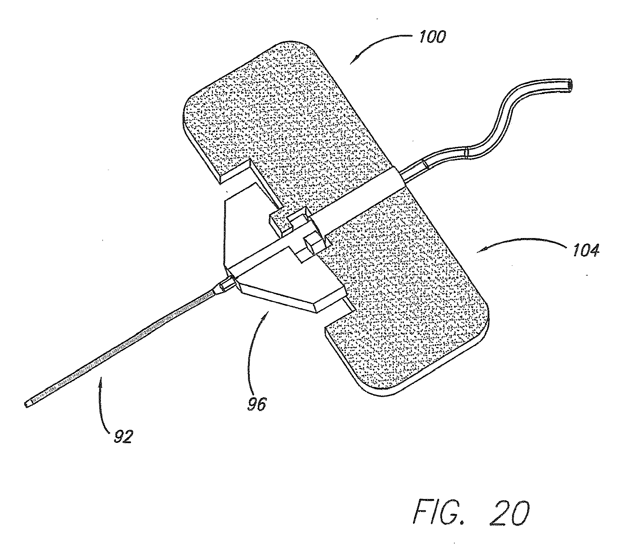

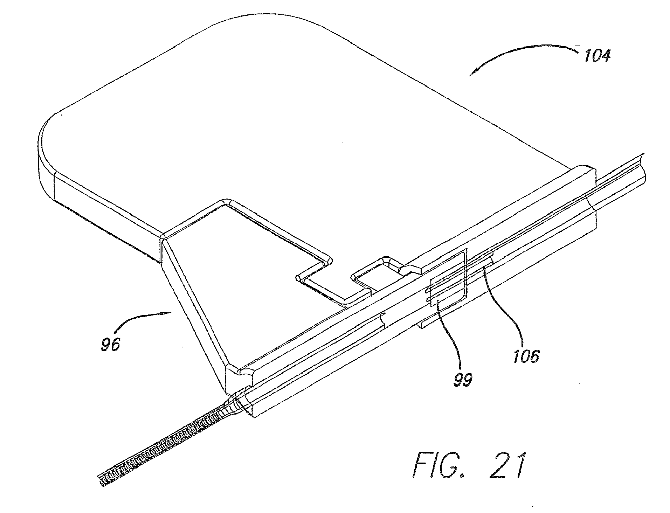

FIG. 20 illustrates an optional feature of the attachable system 90, wherein following the initial locking of the infusion set 92 to the conduit 100, a safety feature of the system 90 indicates to the user if and when the locking relationship between the infusion set wing 96 and the conduit wing 104 is interrupted. In one embodiment, the safety feature is an alteration in the color of either the infusion set wing or the conduit wing to indicate to the user that the components are unlocked and have the potential to detach from one another. Other types of indicators/safety features are also possible, including, for example, an alarm mechanism that produces an audible sound upon unlocking, a temperature mechanism that produces a heated or cooled component upon unlocking, etc. FIG. 21 is a cross-sectional view of the locked assembly, showing in detail the insertion of the cannula 106 through the septum 99, such that fluids can be delivered from the conduit 100 to the infusion set 92.

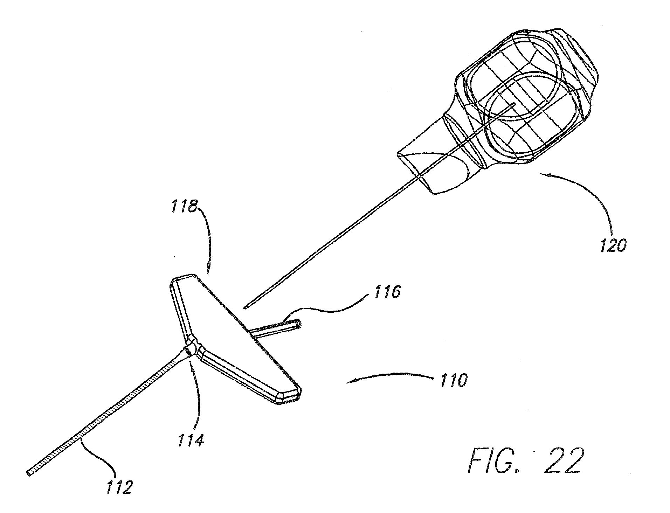

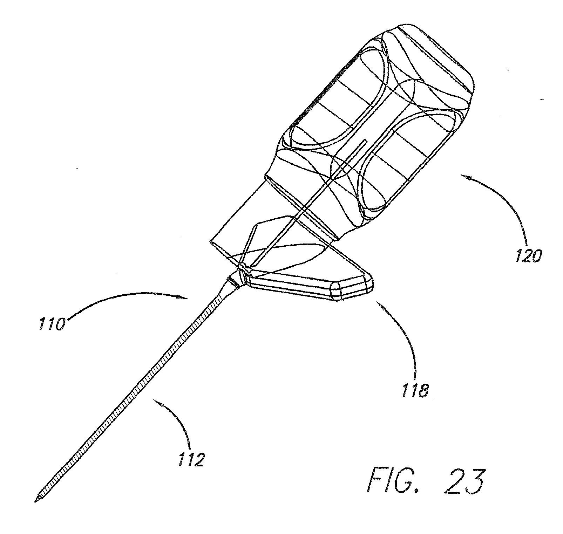

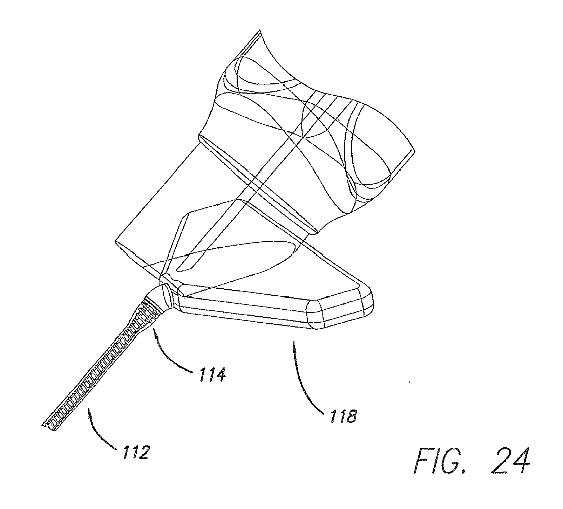

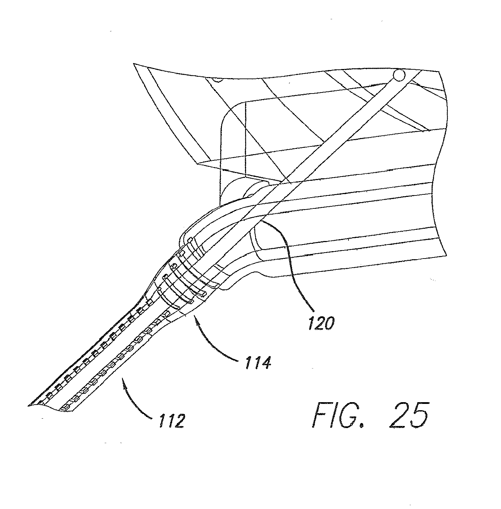

FIGS. 22-25 illustrate an integral infusion set and conduit device, which incorporates certain aspects discussed above. The device 110 shown in FIGS. 22-25 includes a coil-reinforced cannula 112 with a proximal funnel 114 that is in fluid communication with a conduit tubing 116 via a winged hub 118 with a throughgoing lumen. Pictured above the device 110 is an insertion needle 120 for positioning the device in a desired location through the user's skin. FIG. 22 illustrates the integral device 110 prior to insertion into the body, with the winged hub 118 being positioned at an angle with respect to the cannula 112. The proximal funnel 114 of the cannula and the self-sealing material of the winged hub 118 permit such angled positioning for insertion, which is advantageous as it appears less intimidating to the user and enhances user comfort. FIG. 23 illustrates the insertion needle disposed through the winged hub 118 and cannula 112, the entire device 110 thus being ready for insertion into a user. FIG. 24 is an enlarged view of FIG. 23, while FIG. 25 is a cross-sectional view of FIG. 24, each illustrating the coil-reinforced aspect of the cannula 112, as well as the proximal funnel 114. Following insertion into a user, the insertion needle 120 is withdrawn and the winged hub 118, being flexible, is positioned flat against the user's skin and adhered thereto with tape or other adhesive. As should be readily appreciated, this embodiment is advantageous with respect to user comfort and ease of use.

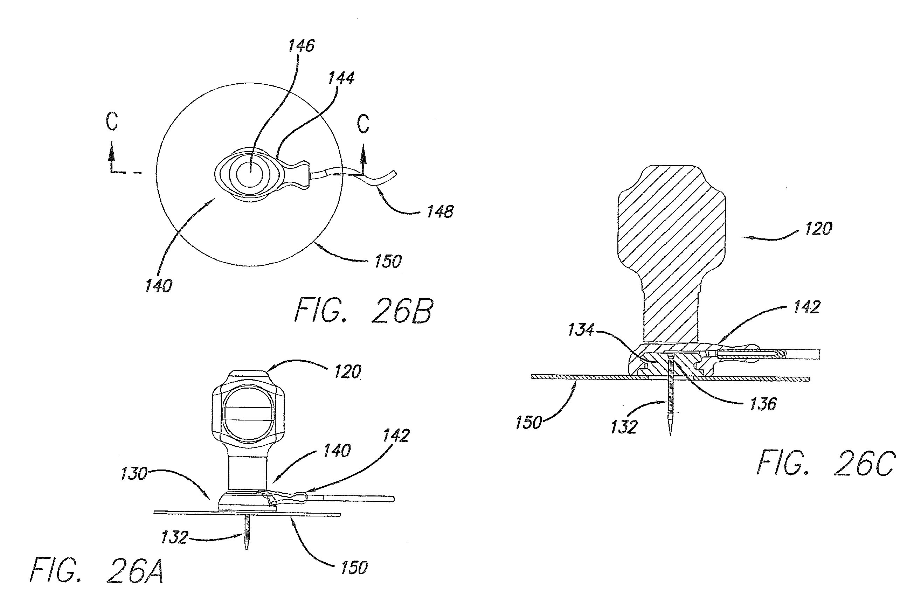

FIGS. 26A-C illustrate another embodiment of an attachable infusion set and conduit assembly. In this embodiment, the cannula 132 of the infusion set 130 is as described herein (e.g., coil-reinforced tubing with proximal funnel), but the cannula hub 134 of the infusion set 130 is shaped to cooperate with a cover portion 142 of the conduit 140 (FIG. 26C). In one embodiment, the assembly components are individually molded, while in another embodiment, the cover portion 142 is overmolded onto the cannula hub 134. The cannula hub 134 may be made of a polymer/elastomer material, for example, while the cover portion 142 may be made of two materials, a high durometer polymer/elastomer outer portion 144 for contact with the cannula hub and a low durometer silicone (or like material) top portion 146 that will self-seal upon removal of the needle following insertion of the infusion set/conduit (FIG. 26B). The cover portion 142 is configured to snap over the cannula hub 134, such that an audible sound and/or tactile sensation is produced to indicate locking connection of the conduit to the infusion set.

As shown in FIG. 26B, a proximal funnel 136 of the cannula 132 is flush with the top of the cannula hub 134, although in other embodiments the cannula hub includes a passageway through the top thereof for fluidly connecting the cannula 132 which has a proximal end positioned below the top surface of the cannula hub 134. Attached to the base of the cannula hub 134, surrounding the cannula 132, is an adhesive patch 150, which is shown in a traditional profile (e.g., circular) for adults, but may instead be configured in a profile that would appeal to children (e.g., lions, tigers, bears, dinosaurs, sea creatures, cartoon characters, etc.). Moreover, the adhesive patch 150 in some embodiments includes one or more of a variety of skin tones or colors to appeal to a variety of age types and user preferences.

Referring to Section C-C of FIG. 26B, the cover portion 142 of the conduit 140 is connected to a tubing portion 148 thereof and has a lumen connecting the tubing portion to a central region that fluidly communicates with the top of the cannula hub 134. The cover portion 142, when attached to the cannula hub 134, forms a seal therewith and is capable of 360 degree rotation. In operation, fluid traveling through the tubing portion 148 and into the lumen of the cover portion 142 of the conduit 140 enters through the proximal end of the cannula (or an opening in the top of the cannula hub and then through the proximal end of the cannula), travels through the cannula 132 and into the body of the user. An insertion needle 120 is shown inserted through the top portion 146 of the cover portion 142 and the cannula 132 for insertion of the assembly into the user.

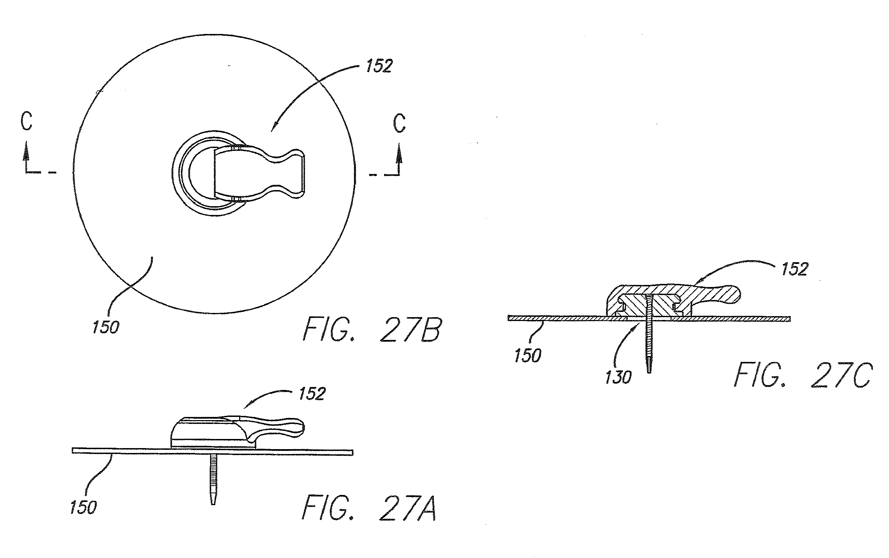

In one embodiment, a safety feature is provided for the infusion set and conduit assembly of FIG. 26. Illustrated in FIG. 27A-C is an activity cover 152, which is similar to the cover portion 142 of the conduit 140 in that it is formed to create a sealing connection with the cannula hub 134, but which does not include a tubing portion or a lumen. Moreover, the activity cover 152 is made from a single material (e.g., polymer/elastomer) as there is no need for a self-sealing top portion. Such a cover when snapped over the cannula hub 134 creates a barrier to outside contamination, which may otherwise enter into the infusion set 130 during certain activities such as bathing, swimming, etc.

### Methods for Manufacturing a Coil-Reinforced Cannula

As mentioned above, there are many different manufacturing methods for creating a coil-reinforced cannula, some of which will be described herein, including associated tipping methods. While specific manufacturing and tipping techniques are discussed herein with particular respect to one another, it should be appreciated that the described techniques may be interchangeable (i.e., each manufacturing technique could be used with each tipping technique). Moreover, the use of the term "core pin" refers to any solid or hollow instrument used in fashioning the coil-reinforced cannula. Thus, "core pin" could mean a mandrel or other tooling instrument that is withdrawn following manufacture of the finished cannula product, or could mean a needle, stylet or other instrument that is intended to become a part of the finished cannula product (but which will be removed following insertion of the coil-reinforced cannula into the user).

In one embodiment, a method for forming a coil-reinforced cannula is a heat shrink body technique, in which a coil component is loaded over (i.e., positioned around) a core pin, after which expanded heat shrink material (e.g., thermoplastic or thermoset) is loaded over the coil component. This assembly is heated, causing the heat shrink material to shrink over the coil component and core pin. Tipping using this technique is dependent on the heat shrink material utilized. Thus, for example, when a thermoplastic material is used (e.g., Pebax™), an RF flashless tip technique, as explained in detail in USPN 4,661,300, which is incorporated by reference herein, may be employed. Conversely, when a thermoset material is used, the tip may be formed using a mechanical technique (e.g., abrasion).

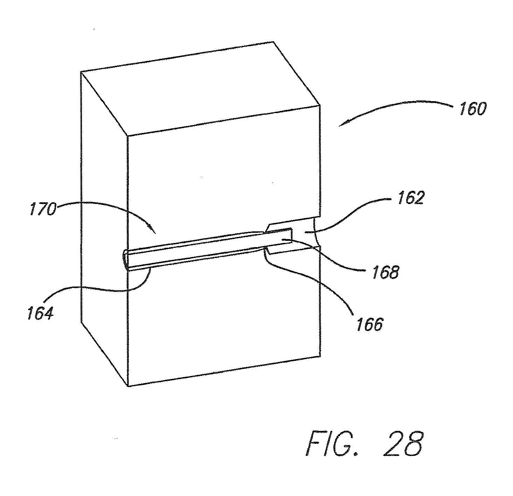

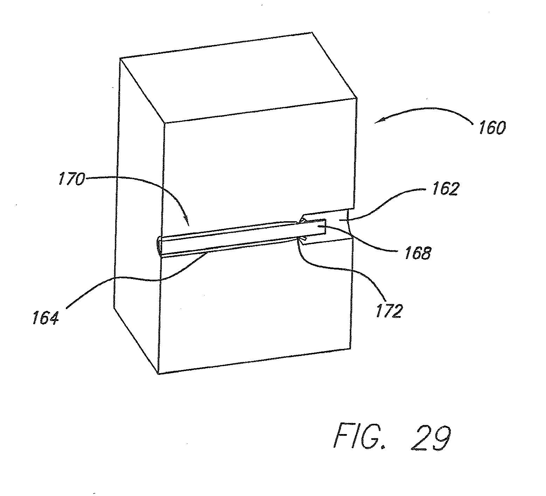

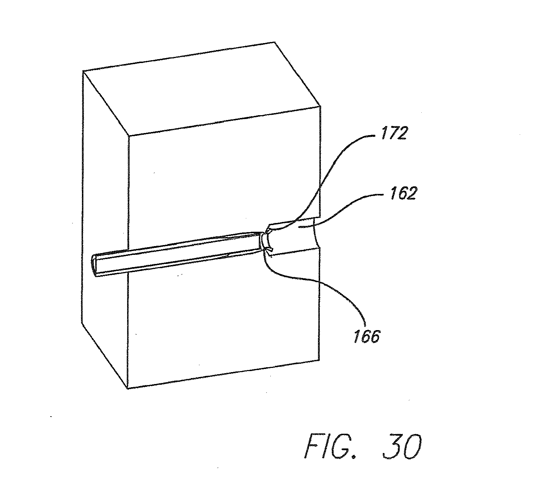

In another embodiment, a method for forming a coil-reinforced cannula is an RF encapsulated tip technique, in which a coil component is first loaded over a core pin, after which thermoplastic tubing is loaded over the coil component. This assembly is then loaded into a tip-forming die. In addition to the tipping techniques mentioned above, an RF stretched neck tear technique may be employed as illustrated in FIGS. 28-30. In FIG. 28, a core pin 168 is shown extending into a wide section 162 of a die 160, with the assembly 170 (e.g., tubing and coil component) surrounding the core pin 168 in the narrow section 164 of the die 160. The die 160 contains a neck-down section 166 at the junction of the wide and narrow sections. In FIG. 29, the assembly 170 is shown pressed through the die 160 toward the wide section 162 thereof such that excess tubing material 172 of the assembly 170 is within the wide section 162. FIG. 30 shows withdrawal of the core pin 168 from the wide section 162 into the narrow section 164 after the tip has been formed, leaving the excess tubing material 172 in the wide section 162 as the neck-down section acts to separate the tubing material 172 from the assembly 170. Using this technique, the distal end of the coil component of the assembly 170 may be embedded into the wall of the tip section thereof.

In another embodiment, a method for forming a coil-reinforced cannula is an RF infiltrated technique, in which a coil component is first loaded over a core pin, after which thermoplastic tubing is loaded over the coil component. This assembly is then inserted into a heated tip-forming die, having a proximal end that is large enough to accommodate the outside diameter of the thermoplastic tubing. In the heated area, the die necks down to an inside diameter that forces the tubing to melt into the interstitial spaces of the coil component as the assembly is advanced into the die. Tipping procedures can be the same as those mentioned above or others known to one of ordinary skill in the art.

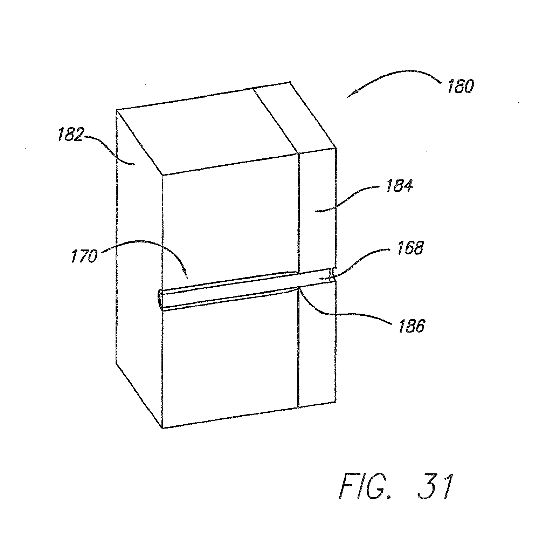

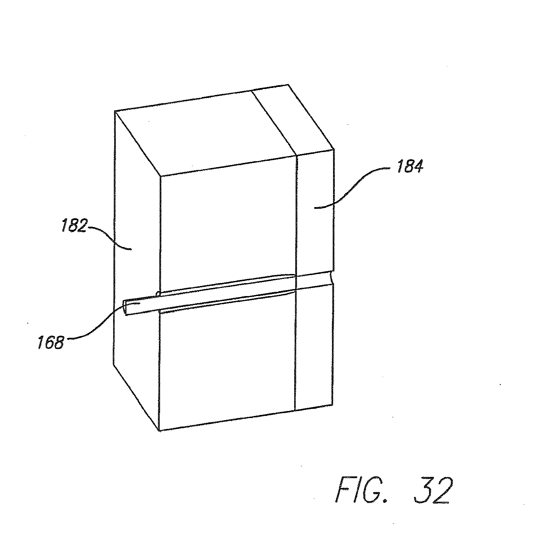

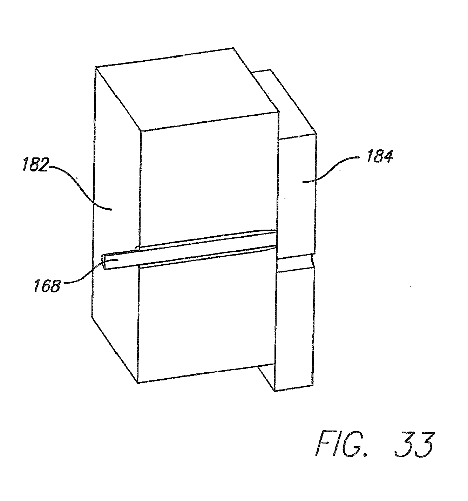

In another embodiment, a method for forming a coil-reinforced cannula is an injection molded infiltrated technique. In this technique a coil component is loaded over a core pin and the assembly is inserted into a wide portion of a die cavity, proximal to a narrow portion thereof. Injectant is then introduced into the die cavity over the assembly as the assembly is advanced therethrough, such that injectant flows around the coil component into the interstices thereof prior to reaching the narrow portion of the die cavity. In addition to the methods discussed above, tipping using this technique can include an injection molded die sheared tip technique as illustrated in FIGS. 31-33. In FIG. 31, a dual block die 180 is shown, including a first block 182 and a second block 184, the first and second blocks including throughgoing lumens that are initially aligned for insertion of a core pin 168 surrounded by an assembly 170. The first block 182 contains a neck-down section 186 at an end thereof adjacent to the second block 184. In FIG. 31, only the core pin 168 is positioned within the lumen of the second block 184. FIG. 32 shows the core pin 168 withdrawn into the lumen of the first block 182 such that it has an end approximately flush with the distal end thereof. FIG. 33 shows the second block 184 moving with respect to the first block 182, such that any material extending beyond the first block 182 into the lumen of the second block 184 is sheared, leaving a formed tip for the assembly 170. After molding has been completed, the proximal end of the tubing may be trimmed.

In another embodiment, a method for forming a coil-reinforced cannula is an injection molded encapsulated technique, which is similar to the injection molded infiltrated technique, but in which the interstices between the coil component are not necessarily filled and, instead, the coil component is encapsulated by a tubing material. The coil-reinforced cannula is then tipped according to any of the techniques described above or others known to one of ordinary skill in the art. In another embodiment, a method of forming a coil-reinforced cannula is a dip coating technique in which a coil component is dip or spray coated with a thermoset or solvent dissolved thermoplastic material. The coil-reinforced cannula is then tipped according to any of the techniques described above or others known to one of ordinary skill in the art. In another embodiment, a method of forming a coil-reinforced cannula is a heat shrink infiltrated technique as taught in USPN 6,702,972, which is incorporated by reference herein. The coil-reinforced cannula is tipped according to any of the techniques described above or others known to one of ordinary skill in the art.

### Sterile Package and Insertion Mechanism

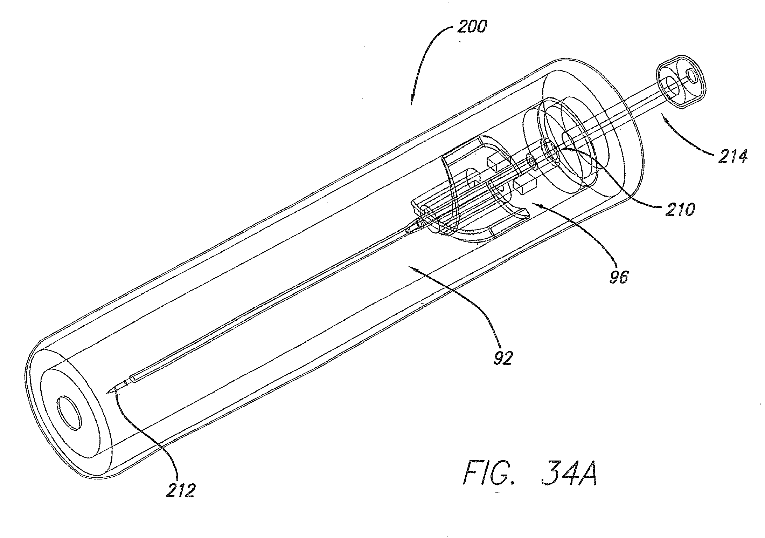

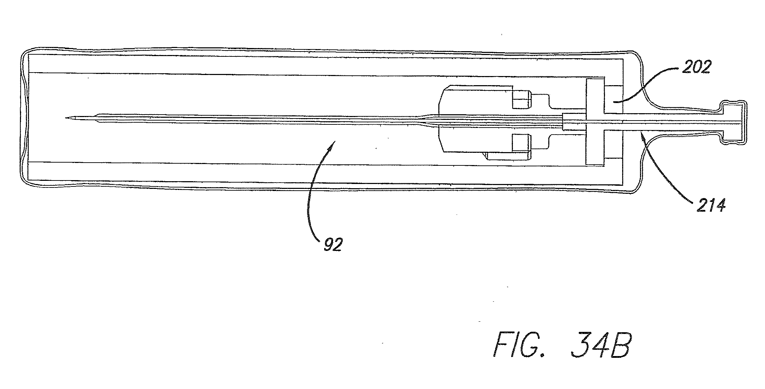

FIGS. 34-37 illustrate an embodiment for a sterile package and insertion mechanism that provides a sterile insertion of an infusion set and a safety mechanism for prevention of accidental needle sticks. While the embodiment shown is with respect to the insertion of an infusion set such as that described above in connection with FIGS. 18-21, it should be appreciated that the principles of this invention would be widely applicable to infusion sets in general and the insertion and packaging device may be fashioned according to the particular configuration of any infusion set or infusion set/conduit combination.

FIGS. 34A-34B illustrate a cartridge with sterile barrier insertion and packaging device according to the present invention. A cartridge 200, which in this embodiment is cylindrical in shape, holds infusion set 92, such that the infusion set wing 96 is folded about a longitudinal axis of the infusion set 92. An insertion needle 210 is positioned through the cannula 94 of the infusion set 92 with a tip 212 of the insertion needle 210 extending beyond the distal tip section of the cannula 94, the insertion needle hub/handle 214 extending through an opening 202 in the proximal end of the cylindrical cartridge 210 (as better seen in FIG. 34B). Initially surrounding the cartridge 200 for shipping and prior to use is a sterile barrier 204 (e.g., aluminum foil, Tyvek^{®}, plastic, etc.). In one embodiment, prior to encasing the infusion set 92 within the cartridge 200, an ointment or gel (e.g., silicone oil) is inserted into the cartridge 200 for lubrication purposes. In another embodiment a medicant, such as an antibacterial, antiscarring or anesthetic agent (e.g., Neosporin^{®}) is inserted into the cartridge 200. Of course, any combination of these or other substances could be placed within the cartridge 200 prior to insertion of the infusion set 92. -

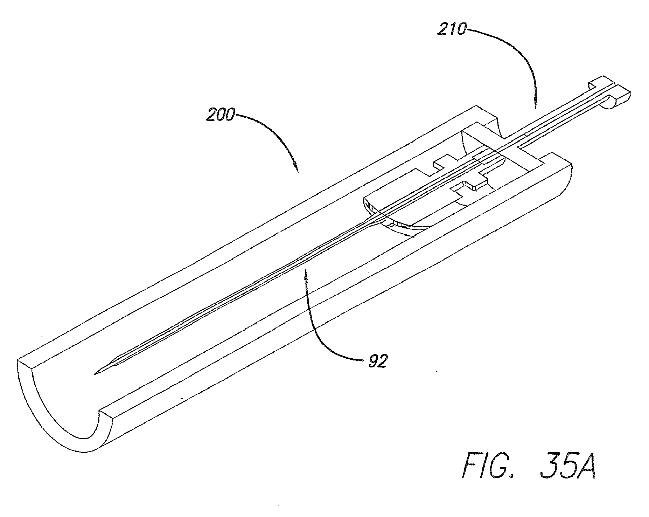

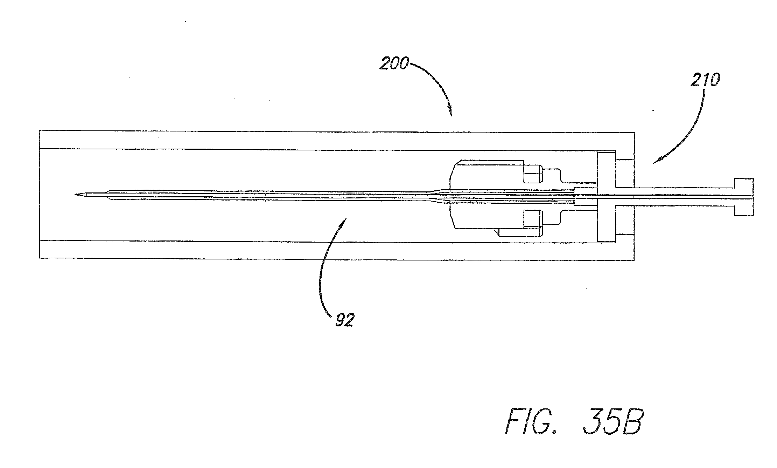

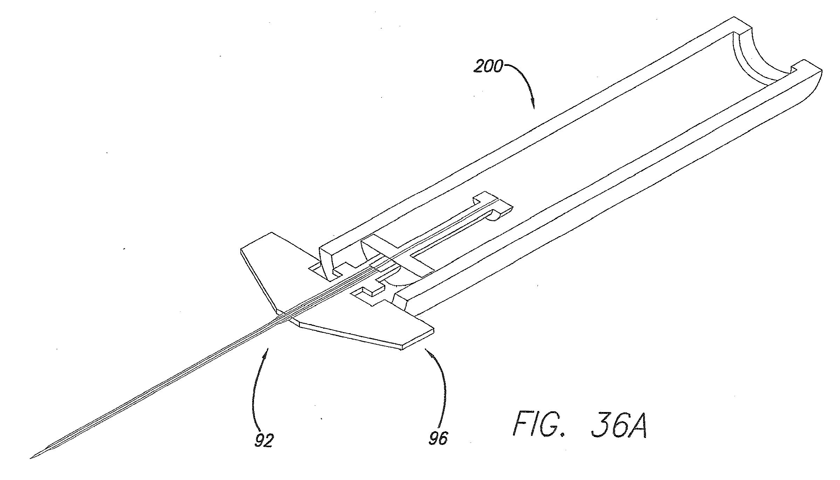

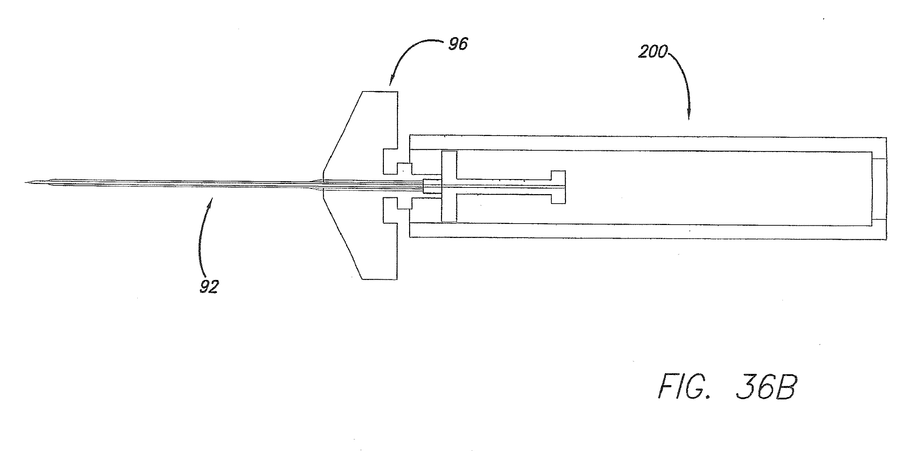

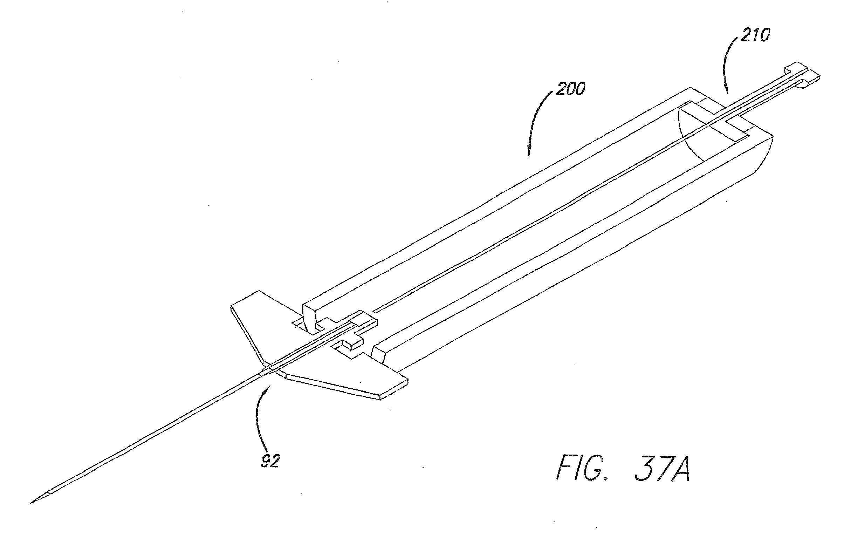

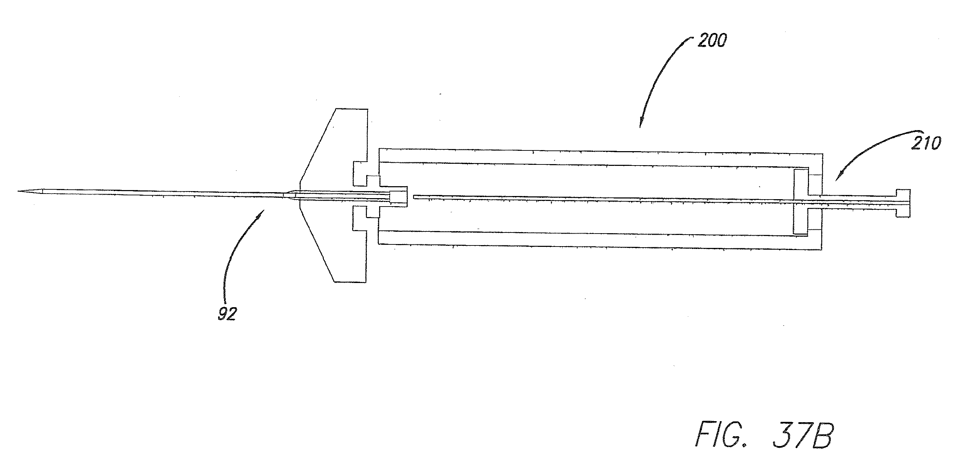

FIGS. 35A-35B show cross-sectional views of the cartridge 200 following removal of the sterile barrier 204, but prior to insertion of the infusion set 92 into a body. It should be noted that the interior of the cartridge 200 continues to provide a sterile field for the infusion set 92 and insertion needle 210 after the barrier 204 is removed. FIGS. 36A-36B show the infusion set 92 as it is ejected from the cartridge 200 and into a user's body. As the infusion set wing 96 exits the cartridge 200, it unfolds from its encased configuration. FIGS. 37A-37B illustrate the safety aspect of the invention as the insertion needle 210 is retracted back into the cartridge 200. The mechanism for such retraction in one embodiment is a spring or similar functioning device, although other retraction mechanisms known to one of ordinary skill in the art are contemplated and are within the scope of the invention.

In a method for inserting an infusion set, according to the embodiment illustrated in FIGS. 34-37, a site on a body of a user is first selected. The sterile barrier 204 is then removed from around the cartridge 210 and insertion needle 210 and the distal end of the cartridge 210 is placed over the selected site on the user's body. The user (or clinician) exerts a force on the needle hub/handle 214 such that the needle tip 212 and cannula 94 of the insertion set 92 exit the distal end of the cartridge 210 and penetrate the user's skin. The force is then removed from the handle 214, causing the needle body and tip 212 to retract back into the cartridge due to the action of the retraction mechanism. A conduit, such as described herein, is then connected to the proximal end of the infusion set 92. The cartridge and insertion needle are discarded.

This invention has been described and specific examples of the invention have been portrayed. While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention, which are within the spirit of the disclosure or equivalent to the inventions found in the claims, it is the intent that this patent will cover those variations as well.
Further disclosure is given in the numbered paragraphs below:
1. A cannula for an infusion set, comprising: a tubing component including an outer wall; and a coil component being arranged to define a lumen, the lumen including a first lumen diameter along a body section of the cannula and a second lumen diameter at a proximal end of the cannula, the second lumen diameter being greater than the first lumen diameter.
2. The cannula according to para. 1, wherein the lumen of the coil component progressively increases in diameter in a proximal direction at the proximal end of the cannula.
3. The cannula according to para. 1, wherein the coil component is continuous and includes helical turns about a longitudinal axis.
4. The cannula according to para. 1, wherein a pitch of the coil component is open or closed along a majority of the length of the cannula.
5. The cannula according to para. 1, wherein the coil component extends distally into a tip of the cannula.
6. The cannula according to para. 1, wherein the coil component is substantially embedded within the wall of the tubing component.
7. The cannula according to para. 1, wherein the coil component includes a corrugated outer surface profile, and wherein the tubing component is positioned over the coil component and attached thereto, the outer surface of the tubing component assuming the outer surface profile of the coil component.
8. The cannula according to para. 1, wherein the outer wall of the tubing component includes a pattern of apertures along a distal length thereof.
9. The cannula according to para. 8, wherein the diameter of the apertures is in the range of approximately 0.0005 in. to 0.020 in.
10. The cannula according to para. 8, wherein the diameter of the apertures is in the range of approximately 0.001 in. to 0.012 in.
11. The cannula according to para. 1, wherein the lumen of the tubing component includes a non-kinking cross-sectional shape.
12. An assembly for subcutaneous delivery of fluid to a body, comprising: an infusion set including an infusion set cannula and an infusion set hub, the infusion set hub including a proximal member that includes a self-sealing member; and a conduit including a conduit hub, the conduit hub including a conduit cannula positioned within the conduit hub to penetrate the re- sealable member upon attachment of the infusion set to the conduit and a conduit opening configured to mate with the proximal member of the infusion set.
13. The assembly according to para. 12, wherein the infusion set cannula includes a coil-reinforced section.
14. The assembly according to para. 12, further comprising a locking feature to lock the infusion set to the conduit.
15. The assembly according to para. 14, further comprising an indicator feature that indicates to a user when the locking interface between the infusion set and the conduit is interrupted.
16. A device for subcutaneous delivery of fluid to a body, comprising: a cannula including a coil component arranged along a length of the cannula to define a lumen, the lumen at a proximal end of the cannula progressively increasing in diameter in a proximal direction; and a hub including a self-sealing material.
17. An assembly for subcutaneous delivery of fluid to a body, comprising: an infusion set including a cannula and a hub, the cannula including a coil component arranged along a length of the cannula to define a lumen, the cannula also including a proximal section positioned within the hub; a conduit including a cover portion shaped to provide a locking interference fit with the hub when attached thereto, the cover portion including a lumen to fluidly connect a tubing portion of the conduit to the cannula; and an adhesive patch connected to the hub.
18. The assembly according to para. 17, wherein the cover portion is fully rotatable with respect to the infusion set hub when attached thereto.
19. The assembly according to para. 17, the cover portion of the conduit including a self-sealing region to permit passage of an insertion needle therethrough.
20. A kit, comprising the assembly according to para. 17 and an activity cover shaped to provide a locking interference fit with the hub when attached thereto following detachment of the conduit.
21. A package and insertion mechanism for a subcutaneous infusion set, comprising: a cartridge configured to accommodate an infusion set, the cartridge including a proximal opening and a distal opening; an insertion needle including a handle extending through the proximal opening of the cartridge; and a sterile barrier positioned around the cartridge and insertion needle handle.
22. The package according to para. 21, further comprising a lubricating fluid positioned within the cartridge.
23. The package according to para. 21, further comprising a medicant positioned within the cartridge.
24. The package according to para. 21, further comprising a retraction mechanism coupled to the insertion needle to retract a tip of the needle into the cartridge following ejection of the infusion set from the cartridge.
25. A method of inserting an infusion set, comprising: selecting a site on a body; removing a sterile barrier from around a cartridge and an insertion needle, the cartridge including an infusion set, the insertion needle including a handle extending from a proximal end of the cartridge and a needle body disposed within the infusion set in the cartridge; positioning a distal end of the cartridge against the selected body site; exerting a force on the insertion needle handle such that a needle tip and cannula portion of the infusion set enter the body at the selected site; and removing the insertion needle from the infusion set, the needle tip retracting into the cartridge.
26. The method according to para. 25, wherein the insertion needle is biased in a pre-insertion position, the removing step including removing a force exerted on the insertion needle handle.
27. The method according to para. 25, further comprising the step of attaching a conduit to the proximal end of the infusion set following removal of the insertion needle.
28. A method of forming a cannula for an infusion set, comprising: loading a coil component over a core pin, the coil component including a first lumen diameter and a second lumen diameter, the second lumen diameter being located at a first end of the coil component and being greater than the first lumen diameter; and loading a tubing component over the coil component.
29. The method according to para. 28, wherein the step of loading a tubing component over the coil component comprises positioning the coil component and core pin into a die and introducing injectant into the die.
30. The method according to para. 28, further comprising the step of forming a tip on an end of the assembly opposite the first end of the coil component.

## Claims

1. An assembly for subcutaneous delivery of fluid to a body, comprising: an infusion set including an infusion set cannula and an infusion set hub, the infusion set hub including a proximal member that includes a self-sealing member; and a conduit including a conduit hub, the conduit hub including a conduit cannula positioned within the conduit hub to penetrate the re- sealable member upon attachment of the infusion set to the conduit and a conduit opening configured to mate with the proximal member of the infusion set.

2. The assembly according to claim 1, wherein the infusion set cannula includes a coil-reinforced section.

3. The assembly according to claim 1 or claim 2, further comprising a locking feature to lock the infusion set to the conduit.

4. The assembly according to claim 3, further comprising an indicator feature that indicates to a user when the locking interface between the infusion set and the conduit is interrupted.

5. An assembly for subcutaneous delivery of fluid to a body, comprising: an infusion set including a cannula and a hub, the cannula including a coil component arranged along a length of the cannula to define a lumen, the cannula also including a proximal section positioned within the hub; a conduit including a cover portion shaped to provide a locking interference fit with the hub when attached thereto, the cover portion including a lumen to fluidly connect a tubing portion of the conduit to the cannula; and an adhesive patch connected to the hub.

6. The assembly according to claim 5, wherein the cover portion is fully rotatable with respect to the infusion set hub when attached thereto.

7. The assembly according to claim 5 or claim 6, the cover portion of the conduit including a self-sealing region to permit passage of an insertion needle therethrough.

8. A kit, comprising the assembly according to any one of claims 5 to 7 and an activity cover shaped to provide a locking interference fit with the hub when attached thereto following detachment of the conduit.

9. An assembly for subcutaneous delivery of a fluid to a body according to any of claims 1 to 8, and including a package and insertion mechanism for a subcutaneous infusion set, comprising:
a cartridge configured to accommodate an infusion set, the cartridge including a proximal opening and a distal opening; an insertion needle including a handle extending through the proximal opening of the cartridge; and a sterile barrier positioned around the cartridge and insertion needle handle.

10. The package according to claim 9, further comprising a lubricating fluid positioned within the cartridge.

11. The package according to claim 9 or claim 10, further comprising a medicant positioned within the cartridge.

12. The package according to any of claims 9 to 11, further comprising a retraction mechanism coupled to the insertion needle to retract a tip of the needle into the cartridge following ejection of the infusion set from the cartridge.

13. The assembly according to any of the preceding claims, wherein the cannula of the infusion set comprises:
a tubing component including an outer wall; and
a coil component being arranged to define a lumen, the lumen including a first lumen diameter along a body section of the cannula and a second lumen diameter at a proximal end of the cannula, the second lumen diameter being greater than the first lumen diameter,
and preferably wherein the lumen of the coil component progressively increases in diameter in a proximal direction at the proximal end of the cannula,
and preferably wherein the coil component is continuous and includes helical turns about a longitudinal axis, the pitch of the coil component being open or closed along a majority of the length of the cannula.

14. The assembly according to claim 13, wherein the coil component extends distally into a tip of the cannula,
and preferably wherein the coil component is substantially embedded within the wall of the tubing component,
and wherein preferably the coil component includes a corrugated outer surface profile and the tubing component is positioned over the coil component and attached thereto, the outer surface of the tubing component assuming the outer surface profile of the coil component,
and wherein preferably the lumen of the tubing component includes a non-kinking cross-sectional shape,
and wherein preferably the outer wall of the tubing component includes a pattern of apertures along a distal end thereof, the diameter of the apertures being preferably in the range of approximately 12.5 µm to 500 µm (0.0005 in. to 0.020 in.) and wherein preferably the diameter of the apertures is in the range of approximately 25 µm to 300 µm (0.001 in. to 0.012 in.).

15. A device for subcutaneous delivery of fluid to a body, comprising: a cannula including a coil component arranged along a length of the cannula to define a lumen, the lumen at a proximal end of the cannula progressively increasing in diameter in a proximal direction; and a hub including a self-sealing material.
